# EUROPÄISCHE PATENTSCHRIFT

(11) **EP 1 966 594 B1**
(45) Veröffentlichungstag und Bekanntmachung des Hinweises auf die Patenterteilung: **23.01.2013**
(21) Anmeldenummer: 06829574.0
(22) Anmeldetag: 13.12.2006
(51) Int. Cl.: G01N 21/64, G01N 21/77, G01N 33/543, G01N 33/569

(54) **VORRICHTUNG UND VERFAHREN ZUM NACHWEIS VON MYKOTOXINEN**
DEVICE AND METHOD FOR IDENTIFYING MYCOTOXINS
PROCEDE ET DISPOSITIF POUR PROUVER LA PRESENCE DE MYTOTOXINES

(30) Priorität: 23.12.2005 DE 102005062377
(43) Veröffentlichungstag der Anmeldung: 10.09.2008
(73) Patentinhaber: Bayer Intellectual Property GmbH, 40789 Monheim (DE); Bayer CropScience Aktiengesellschaft, 40789 Monheim (DE)
(72) Erfinder: BURMEISTER, Jens, 51375 Leverkusen (DE); DORN, Ingmar, 50668 Köln (DE); RABE, Uwe, 51381 Leverkusen (DE); HÄUSER-HAHN, Isolde, 51375 Leverkusen (DE)
(74) Vertreter: BIP Patents
(86) Internationale Anmeldenummer: PCT/EP2006/012000
(87) Internationale Veröffentlichungsnummer: WO 2007/079893

(56) Entgegenhaltungen:
- WO-A-00/43552
- WO-A-02/20873
- LIGLER FRANCES S ET AL: "Array biosensor for detection of toxins." ANALYTICAL AND BIOANALYTICAL CHEMISTRY OCT 2003, Bd. 377, Nr. 3, Oktober 2003 (2003-10), Seiten 469-477, XP002430514 ISSN: 1618-2642
- MARK J FELDSTEIN ET AL: "Array Biosensor: Optical and Fluidics Systems" BIOMEDICAL MICRODEVICES, KLUWER ACADEMIC PUBLISHERS, BO, Bd. 1, Nr. 2, 1. Dezember 1998 (1998-12-01), Seiten 139-153, XP019205048 ISSN: 1572-8781
- NGUNDI MIRIAM M ET AL: "Array biosensor for detection of ochratoxin A in cereals and beverages." ANALYTICAL CHEMISTRY 1 JAN 2005, Bd. 77, Nr. 1, 1. Januar 2005 (2005-01-01), Seiten 148-154, XP002430515 ISSN: 0003-2700 in der Anmeldung erwähnt
- KLOTZ A, BARZEN C, BRECHT A, HARRIS RD, QUIGLEY GR, WILKINSON JS, GAUGLITZ G: 'Sensitivity enhancement of transducers for total internal relfection fluorescence' Bd. 3620, 1999, PROC SPIE, Seiten 345 - 354

## Beschreibung

Die Erfindung betrifft eine Vorrichtung und ein Verfahren zum Nachweis von Mykotoxinen sowie Kits, die zur Durchführung des Verfahrens geeignet sind.

Der Nachweis von Mykotoxinen umfasst ein großes Anwendungsgebiet z.B. im Nahrungs- und Futtermittelbereich, in der Umweltanalytik, im Pflanzenschutz sowie in der biochemischen Forschung.

Mykotoxine sind von Schimmelpilzen gebildete Giftstoffe mit sehr unterschiedlicher chemischer Struktur. Mykotoxine kommen in Ernteprodukten wie Getreide, ölhaltigen Samen und Früchten vor und können Ursache von Vergiftungen bei Mensch und Tier sein. Inzwischen sind über 300 verschiedene Mykotoxine identifiziert worden, die in ca. 25 Strukturtypen eingeteilt werden und unterschiedliche toxische Wirkungen zeigen. Mykotoxine können, je nach Toxinart, akute oder chronische Vergiftungen hervorrufen. Häufig vorkommende Gruppen von Mykotoxinen sind Aflatoxine, Ochratoxine, Mutterkornalkaloide, Patulin und Fusarientoxine. Unter den Fusarientoxinen sind-Deoxynivalenol, Zearalenon, Nivalenol, T-2-/HT2-Toxin und die Fumonisine von besonderer Bedeutung, da sie häufig in Getreideerzeugnissen vorkommen. Entsprechend sollte ein Test auf Mykotoxine, beispielsweise auf Toxine von Feldpilzen, z.B. Fusarientoxine, oder auf Toxine von Lagerpilzen an den Getreideannahmestellen, bei Getreidehändlern sowie Getreideverarbeitem, beispielsweise Mühlen, Mälzereien, Futtermittelhersteller, bei Landwirten, Beratungsstellen, Universitäten oder Ministerien beispielsweise dem Ministerium für Verbraucherschutz durchgeführt werden, um die Qualität der Nahrungsmittel sicherzustellen.

Im Stand der Technik sind eine Reihe von Verfahren zum Nachweis von Mykotoxinen bekannt. Der Nachweis von Mykotoxinen erfolgt zum Beispiel durch chromatographische Verfahren wie HPLC, die mit fluoreszenzbasierter, absorptiver oder massenspektrometrischer Detektion gekoppelt sein kann. Vor der HPLC-Analyse, beispielsweise einer Getreide-Probe, erfolgt in der Regel die Anreicherung und Aufreinigung des Analyten mittels Immuno-Affinitäts-Säulen. Nachteile aller HPLC-basierten Verfahren sind die hohen Investitionskosten, die relativ komplexe Probenhandhabung sowie lange Analysezeiten. Aufgrund der genannten Nachteile sind HPLC-basierte Nachweisverfahren nicht zur schnellen, billigen und einfachen Analyse, beispielsweise von Getreideproben in Getreide erzeugenden, annehmenden, handelnden oder verarbeitenden Betrieben geeignet. Stattdessen erfolgt die HPLC-basierte Analyse in spezialisierten, analytischen Labors. In der Praxis steht das Ergebnis dementsprechend erst mit einer Zeitverzögerung von mehreren Tagen zur Verfügung.

Eine alternative Methode zum Nachweis von Mykotoxinen ist die ELISA-Technik (Enzyme Linked Immuno Sorbent Assay). Für den ELISA-Test werden Mikrotiterplatten bereitgestellt, deren Vertiefungen beispielsweise mit Fänger-Antikörpern beschichtet sind, die spezifisch an ein Mykotoxin binden. Nachteile des ELISA-Tests sind die vielen Pipettier-, Wasch- und Inkubationsschritte, die zu relativ langen Analysezeiten von mehr als 30 Minuten führen können. Dies verhindert es, den Test schnell vor Ort außerhalb eines analytischen Labors durchzuführen. Zudem erlaubt der Test nicht den gleichzeitigen Nachweis mehrerer Analyten, da in der Regel jede Mikrotiterplatte nur mit einer Sorte von Antikörpern beschichtet ist.

Eine weitere Methode zum Nachweis von Mykotoxinen sind Lateral Flow Assays (LFA). Zum Nachweis von Mykotoxinen mittels LFA kann beispielsweise ein direkter, kompetitiver Immunoassay auf einem Nitrozellulosestreifen ausgeführt werden, wobei die zu analysierende Probe aufgrund von Kapillarkräften durch den gesamten Nitrozellulosestreifen durchgezogen wird. Nachteilig ist, dass das Verfahren nur einen qualitativen Mykotoxin-Nachweis erlaubt. Weiterhin nachteilig ist, dass für jedes Mykotoxin bei diesem Test ein separater Streifen benötigt wird.

Im Stand der Technik finden sich auch Arbeiten zur Entwicklung von Nachweisverfahren für Mykotoxine, beispielsweise beschrieben von M. M. Ngundi et al., Anal. Chem. 2005, 77, 148-154. In diesem Verfahren wird ein indirekt, kompetitiver Immuno-Assay zum Nachweis von Ochratoxin A ausgeführt, indem Ochratoxin A auf Glasobjektträgern immobilisiert wird. Das Gemisch eines fluoreszenzmarkierten Antikörpers gegen Ochratoxin A und der zu bestimmenden Probe wird auf den Glasobjektträger gegeben und nach Entfernen der nichtgebundenen Antikörper durch Waschen kann dieser ausgelesen werden. Nachteilig bei diesem Verfahrens ist, dass Waschschritte und Inkubationszeiten von 10 bis 20 Minuten benötigt werden und aufwändige Fluoreszenz-Imaging-Syteme zum Auslesen der Ergebnisse erforderlich sind. Dieses ermöglicht es nicht, einen vor Ort durchführbaren Schnelltest außerhalb eines analytischen Labors auf dieser Grundlage zu entwickeln.

F. S. Ligler et al., Analytical and Bioanalytical Chemistry, 2003, 377, 3, 469-477 beschreibt eine Methode zur Analyse von Mykotoxinen mittels Wellenleiter-Array-Sensor in einem kompetitiven Fluoroimmunoassay. Um das Mykotoxin Fumonisin zu bestimmen, wurde ein kompetitiver Assay gewählt, wo Fumosinin an die Sensoroberfläche immobilisiert wird und mit einem Gemisch aus fluoreszenzmarkiertem Cy5-anti-Fumonisin-Antikörper und Probe inkubiert wird.

Die im Stand der Technik bekannten Verfahren zum Nachweis von Mykotoxinen benötigen somit hohe Investitionskosten aufgrund komplizierter Auslesegeräte, beinhalten viele manuelle Schritte, oder sind nicht außerhalb eines analytischen Labors einsetzbar.

Der vorliegenden Erfindung liegt daher die Aufgabe zugrunde, ein Verfahren zur Verfügung zu stellen, das wenigstens einen der vorgenannten Nachteile des Standes der Technik überwindet, insbesondere, ein Verfahren bereitzustellen, das schnell, einfach handhabbar und kostengünstig den Nachweis von Mykotoxinen in einer Probe ermöglicht.

Diese Aufgabe wird gelöst durch ein Verfahren zum Schnell-Nachweis von Mykotoxinen umfassend folgende Schritte:

Weiters beschrieben ist eine Vorrichtung zur Durchführung des Verfahrens zum Nachweis von Mykotoxinen.

Ein weiterer Gegenstand ist ein Kit geeignet zur Durchführung des Verfahrens zum Nachweis von Mykotoxinen.

Weitere vorteilhafte Ausgestaltungen der Erfindung ergeben sich aus den Unteransprüchen.

Überraschend wurde gefunden, dass das erfindungsgemäße Verfahren zum Nachweis von Mykotoxinen einfach durchzuführen ist, und außerhalb spezialisierter Analyselabors durchgeführt werden kann. Dies ermöglicht es, dass das erfindungsgemäße Verfahren in Form eines Schnelltests durchgeführt werden kann, ohne dass Proben notwendigerweise in ein Labor zur Analyse gegeben werden müssen. Weiterhin vorteilhaft ist, dass der Mykotoxinnachweis gemäß dem erfindungsgemäßen Verfahren lediglich wenige oder keine Waschschritte benötigt. Dies ist insbesondere von Vorteil, da die Durchführung von Waschschritten zeitaufwändig ist, den Erhalt eines Analyseergebnisses verzögert, und insbesondere bei wenig sorgfältiger oder unsachgemäßer Durchführung die Analyseergebnisse verzerren oder den Nachweis gänzlich unmöglich machen kann.

Die Kombination der vorteilhaften Eigenschaften des erfindungsgemäßen Verfahrens ermöglicht es, dass ein Nachweis von Mykotoxinen in Nahrungsmitteln beispielsweise an Getreideannahmestellen, bei Getreidehändlern oder Getreideverarbeitern durchgeführt werden kann. Insbesondere
a) Bereitstellen eines Dünnschichtwellenleiters umfassend eine erste optisch transparente wellenleitende Schicht (a) auf einer zweiten optisch transparenten Schicht (b), wobei (b) einen niedrigeren Brechungsindex besitzt als (a), und wobei auf Schicht (a) Mykotoxin- Rinderserumalbumin - Konjugate als chemisches oder biochemisches Erkennungselement für einen Mykotoxin-bindenden Bindungspartner räumlich separiert immobilisiert werden,
b) Aufbringen einer Mykotoxin(e) enthaltenden Probe und von Mykotoxin-bindenden Bindungspartnern zu den immobilisierten Mykotoxin-Rinderserumalbumin -Konjugate auf dem Dünnschichtwellenleiter, wobei ein Markierungselement an einen Bindungspartner oder an den immobilisierten Mykotoxin- Rinderserumalbumin -Konjugate gebunden ist,
c) Detektieren eines Signals im Evaneszentfeld, das durch Änderung der optischen Eigenschaften an der Grenzfläche zum Wellenleiter infolge der Wechselwirkung der auf dem Dünnschichtwellenleiter immobilisierten Mykotoxin- Rinderserumalbumin -Konjugate mit den Mykotoxinen aus der Probe und/oder den Mykotoxin-bindenden Bindungspartnern hervorgerufen wird,
dann
d) Bestimmen der in der Probe vorliegenden Menge an Mykotoxin(en).
ermöglicht die einfache und schnelle Durchführbarkeit des Verfahrens, dass dieses auch von Personen durchgeführt werden kann, die keine spezialisierten Analytiker eines Fachlabors sind.

In bevorzugten Ausführungsformen des Verfahrens verwendet man als Dünnschichtwellenleiter einen Evaneszent-Feld-Biochip, der auf einem Dünnschichtwellenleiter basiert, vorzugsweise einen planaren optischen Wellenleiter-Biochip, der auf einem Dünnschichtwellenleiter basiert.

Optische Wellenleiter sind eine Klasse von Signalwandlern, mit denen man die Änderung der optischen Eigenschaften eines Mediums detektieren kann, das an eine wellenleitende Schicht, typischer Weise ein Dielektrikum, grenzt. Wird Licht als geführte Mode in der wellenleitenden Schicht transportiert, fällt das Lichtfeld an der Grenzfläche Medium/Wellenleiter nicht abrupt ab, sondern klingt in dem an den Wellenleiter angrenzenden sogenannten Detektionsmedium exponentiell ab. Dieses exponentiell abfallende Lichtfeld wird als evaneszentes Feld bezeichnet. Ändern sich die optischen Eigenschaften des an den Wellenleiter grenzenden Mediums innerhalb des evaneszenten Feldes, kann dies über einen geeigneten Messaufbau detektiert werden.

Vorteilhaft bei der Verwendung von Wellenleitern als Signalwandler ist, dass bei an der Grenzfläche des Wellenleiters immobilisierten Erkennungselementen das Binden an das Erkennungselement oder die Reaktion des Erkennungselementes detektiert werden kann, wenn sich dabei die optischen Eigenschaften des Detektionsmediums an der Grenzfläche zum Wellenleiter ändern.

Entsprechend wird eine Zeitersparnis bei der Durchführung des Nachweises ermöglicht, wie auch eine Vereinfachung des Ablaufs.

Somit kann die Detektion eines Signals bzw. eines markierten Elements über die sich ändernden optischen Eigenschaften des Mediums, beispielsweise einer zu analysierenden Probe, direkt an der Oberfläche des Signalwandlers bzw. Dünnschichtwellenleiters erfolgen, beispielsweise über eine Änderung der Absorption, der Fluoreszenz, der Phosphoreszenz, der Luminseszenz oder ähnlichem.

Vorzugsweise detektiert man im Evaneszentfeld ein Fluoreszenzsignal. Erfindungsgemäß verwendbare Markierungselemente für eine Markierung der Bindungspartner, beispielsweise Mykotoxine, Mykotoxin-Konjugate, Antikörper-Konjugate oder Antikörper sind vorzugsweise organische Fluorophore, Nanopartikel, fluoreszente Nanopartikel, Beads, fluoreszente Beads, fluoreszente Proteine oder andere signalgebende Moleküle oder Einheiten oder beliebige Kombinationen verschiedener Markierungselemente. Bevorzugt verwendet man lumineszenzfähig markierte Bindungspartner. Bevorzugte Markierungselemente sind organische Fluorophore und/oder fluoreszente Proteine.

Gemäß dem erfindungsgemäßen Verfahren kann der vorzugsweise fluoreszente markierte Bindungspartner durch ein evaneszentes Feld angeregt werden. In bevorzugten Ausführungsformen wird das Evaneszente Feld durch einen planaren optischen Wellenleiter, wie in US 5,959,292, Duveneck et al. beschrieben erzeugt. Die isotrop emittierte Fluoreszenz ist durch einen geeigneten Aufbau detektierbar. In anderen Ausführungsformen kann die in den Wellenleiter eingekoppelten Fluoreszenz durch ein geeignetes optisches Element aus dem Wellenleiter wieder ausgekoppelt und mit einem geeigneten optischen Aufbau detektiert werden.

Von besonderem Vorteil ist, dass ein Abwaschen vorzugsweise fluoreszent markierter Bindungspartner oder einer markierte Bindungspartner enthaltenden Probe bzw. Lösung vor der Detektion eines Signals eingeschränkt oder sogar völlig darauf verzichtet werden kann. Dieses ermöglicht eine Zeitersparnis bei einem Mykotoxinnachweis wie auch eine Vereinfachung der Durchführung, da auch auf die Bereitstellung der üblicherweise verwendeten verschiedenen Pufferlösungen des Waschprotokolls verzichtet werden kann.

Verwendbare Dünnschichtwellenleiter umfassen vorzugsweise eine optisch transparente wellenleitende Schicht (a) enthaltend Oxide ausgewählt aus der Gruppe umfassend TiO₂, ZnO, Nb₂O₅, Ta₂O₅, HfO₂ und/oder ZrO₂, bevorzugt ausgewählt aus der Gruppe umfassend TiO₂, Ta₂O₅ und/oder Nb₂O₅. Vorzugsweise ist die optisch transparente wellenleitende Schicht (a) aus TiO₂, ZnO, Nb₂O₅, Ta₂O₅, HfO₂ oder ZrO₂, bevorzugt TiO₂, Ta₂O₅ oder Nb₂O₅ ausgebildet. Insbesondere hat sich die Verwendung von Tantalpentoxid als vorteilhaft herausgestellt, insbesondere bei der Detektion eines Fluoreszenzsignals.

In bevorzugten Ausführungsformen bringt man auf den Dünnschichtwellenleiter, insbesondere auf die optisch transparente wellenleitende Schicht (a) enthaltend Oxide ausgewählt aus der Gruppe umfassend TiO₂, ZnO, Nb₂O₅, Ta₂O₅, HfO₂ und/oder ZrO₂, Mono- oder Mehrfachschichten von Organophosphorsäuren gemäß der nachstehenden Formel (I)

R-OPO₃H₂ (I)

und/oder Organophosphonsäuren gemäß der nachstehenden Formel (II)

R-PO₃H₂ (II)

und/oder deren Salze auf, wobei
- R: steht für Alkyl mit C₁₀ bis C₂₄.

Bevorzugt verwendbar sind Organophosphorsäuren und/oder Organophosphonsäuren, vorzugsweise Organophosphate und/oder Organophosphonate, mit R ist ausgewählt aus der Gruppe umfassend unverzweigtes Alkyl mit C₁₀ bis C₂₀, vorzugsweise ausgewählt aus der Gruppe umfassend unverzweigtes Alkyl mit C₁₂ bis C₁₈, bevorzugt Dodecylphsophorsäure, Dodecylphosphat, Octadecylphosphonat und/oder Octadecylphosphonsäure.

Vorzugsweise sind Organophosphorsäuren bzw. Organophosphate, die in Form wasserlöslicher Salze aus wässriger Lösung auf den Dünnschichtwellenleiter aufgebracht werden können verwendbar.

In bevorzugten Ausführungsformen werden die Organophosphorsäuren und/oder Organophosphonsäuren, vorzugsweise Organophosphate, in Form einer Monoschicht auf den Dünnschichtwellenleiter, insbesondere einen Evaneszent-Feld-Biochip, vorzugsweise einen planaren optischen Wellenleiter-Biochip, aufgebracht. Das Aufbringen kann in Form von Tauchverfahren erfolgen.

Die Monoschicht oder Monolayer kann als eine Haftvermittlungsschicht auf die optisch transparente Schicht ausgebildet aus Oxiden aufgebracht werden. Vorteilhafter Weise können Organophosphorsäuren und/oder Organophosphonsäuren mit Erkennungselementen, insbesondere mit Proteinen oder an Proteine gekoppelten Erkennungselementen wechselwirken und die Bindung der Erkennungselemente an den Biochip verstärken.

Verwendbare Mykotoxin-bindende Bindungspartner sind vorzugsweise ausgewählt aus der Gruppe umfassend Anti-Mykotoxin-Antikörper, Anti-Mykotoxin-Antikörper-Konjugate, Fragmente von Anti-Mykotoxin-Antikörpem, Mykotoxin-bindende Peptide, Mykotoxin-bindende Anticaline, Mykotoxin-bindende Aptamere, Mykotoxin-bindende Spiegelmere und/oder Mykotoxin-bindende imprinted Polymers, bevorzugt ausgewählt aus der Gruppe umfassend Anti-Mykotoxin-Antikörper, Anti-Mykotoxin-Antikörper-Konjugate.

Die Bindungspartner wechselwirken jeweils spezifisch und/oder affin mit den Mykotoxin-Rinderserumalbumin-Konjugaten Beispielsweise binden Anti-Mykotoxin-Antikörper, die auf einem Dünnschichtwellenleiter aufgebracht werden, affin an auf diesem Dünnschichtwellenleiter immobilisierte Mykotoxine. Ebenso binden auf einem Dünnschichtwellenleiter immobilisierte Anti-Mykotoxin-Antikörper affin an Mykotoxine oder Mykotoxin-Konjugate, die auf den Dünnschichtwellenleiter aufgebracht werden. Die Spezifität der Bindung hängt dabei von den verwendeten Affinitätspartnern ab. So binden verwendbare kreuzreaktive Anti-Mykotoxin-Antikörper affin an die entsprechenden Mykotoxine, beispielsweise der Gruppe der Fumosine, aber weniger spezifisch als beispielsweise ein spezieller Antikörper gegen Fumosin B1 wäre. Bindungspartner, die immobilisiert sind, werden auch als Erkennungselement oder "Fängermoleküle" bezeichnet.

Anti-Mykotoxin-Antikörper-Konjugate und Mykotoxin-Konjugate sind beispielsweise ausbildbar aus einem Protein und Anti-Mykotoxin-Antikörper oder Mykotoxin.

In bevorzugten Ausführungsformen beispielsweise bei indirekt kompetitiven Assays sind die immobilisierten Bindungspartner Mykotoxin-Konjugate. Mykotoxin-Konjugate sind vorzugsweise ausbildbar aus Mykotoxin gebunden an Proteine, beispielsweise Rinderserumalbumin (BSA, Bovine Serum Albumine). Von besonderem Vorteil bei der Verwendung eines solchen Mykotoxin-BSA-Konjugats ist, dass eine Verbesserung der Bindung des Mykotoxins an den Dünnschichtwellenleiter über eine Wechselwirkung zwischen Protein und Organophosphorsäuren und/oder Organophosphonsäuren ausgebildet werden kann. Dies kann eine Verbesserung der Haftung der Erkennungselemente an dem Dünnschichtwellenleiter zur Verfügung stellen.

Ein Markierungselement, beispielsweise ein Fluoreszenzfarbstoff oder Fluorophor, kann direkt an einen Bindungspartner, beispielsweise an einen Anti-Mykotoxin-Antikörper oder an ein Mykotoxin gebunden sein, oder über ein spacer-Element, beispielsweise ein Protein oder eine Alkyl- oder Polyethylenglycol-Kette. Das Markierungselement, beispielsweise ein Fluoreszenzfarbstoff oder Fluorophor, ist vorzugsweise über ein Protein an die Mykotoxine gebunden. Ein geeignetes Protein ist beispielsweise BSA. Eine Bindung eines Fluorophors an ein Mykotoxin mittels BSA kann die Bindung des Markierungselements an die Bindungspartner, beispielsweise Antikörper, deutlich verbessern. Ein weiterer Vorteil ergibt sich dadurch, dass aufwändige Verfahren zur direkten Bindung beispielsweise eines Fluorophors an ein Mykotoxin vermieden werden können. Vorzugsweise sind als Bindungspartner für einen immobilisierten Anti-Mykotoxin-Antikörper fluoresenzmarkierte Mykotoxin-BSA-Konjugate verwendbar, beispielsweise in einem direkt kompetitiven Assay.

Der Nachweis der Mykotoxine kann grundsätzlich in auf einem Dünnschichtwellenleiter aufbringbare Proben, Lösungen oder sonstigen Medien erfolgen. In bevorzugten Ausführungsformen sind die Proben Nahrungsmittel für Mensch oder Tier. Bevorzugt erfolgt der Nachweis von Mykotoxinen gemäß dem erfindungsgemäßen Verfahren in Getreide, Getreideprodukten, Wein, Säften oder Früchten, und/oder in Getreide, Wein, Säfte und/oder Früchte enthaltenden Produkte. Hierbei kann die zu analysierende Probe beispielsweise ein Nahrungsmittel oder Produkt auf den Dünnschichtwellenleiter aufgebracht werden oder mit einem Lösemittel oder Lösemittelgemisch extrahiert werden und der extrahierter Extrakt verwendet werden. Der Extrakt kann verdünnt oder konzentriert verwendbar sein.

Die Mykotoxine können aus der zu untersuchenden Probe, zum Beispiel Getreide oder andere Nahrungsmittel, durch Behandlung mit einem Lösemittel oder Lösemittelgemisch herausgelöst werden. Beispielsweise können Mykotoxine aus Getreideproben durch Mahlen und anschließend Extraktion mit Wasser oder organischen Lösemitteln oder Lösemittelgemischen, beispielsweise mit Gemischen aus Wasser, das gegebenenfalls mit Puffersubstanzen, Salzen, Säuren oder Basen und anderen Zusätzen versetzt sein kann, und organischen Lösemitteln, beispielsweise mit Gemischen aus Wasser und Methanol oder Ethanol oder Wasser und Acetonitril herausgelöst werden. Andere Verfahren, die zur Extraktion der Mykotoxine führen, sind dem Fachmann bekannt. Die erhaltenen, gelösten Mykotoxine können anschließend direkt oder nach Verdünnung oder Anreicherung auf dem Dünnschichtwellenleiter oder Chip analysiert werden.

Verwendbare Erkennungselemente, die auch als "Fängermoleküle" bezeichnet werden, vorzugsweise Mykotoxin-BSA-Konjugate, vorzugsweise zwei oder mehrere verschiedene, können auf der Dünnschichtwellenleiter- oder Chip-Oberfläche kovalent oder nichtkovalent, beispielsweise durch hydrophobe Adsorption, immobilisiert werden. Ein Immobilisieren kann beispielsweise durch ein als Spotting bezeichnetes Aufbringen der Erkennungselemente in Form von Messfeldern, sogenannten Spots, auf die Dünnschichtwellenleiter- oder Chipoberfläche erfolgen. Vorzugsweise erfolgt ein Spotting von Lösung vorzugsweise von Pufferlösungen, den das oder die Bindungspartner als Erkennungselement enthalten, durch Geräte zum automatischen Aufbringen, sogenannte Spotter. Vorzugsweise werden die Dünnschichtwellenleiter oder Chips nach dem Spotten wenigstens eine Stunde, vorzugsweise einige Stunden inkubiert, so dass die Erkennungselemente auf dem Dünnschichtwellenleiter oder Chip anheften können.

Es ist bevorzugt, dass die Biochips nach dem Spotten für wenigstens eine Stunde, vorzugsweise 2 Stunden bis 6 Stunden, besonders bevorzugt 3 Stunden bis 4 Stunden mit einer Proteinlösung, vorzugsweise einer Lösung eines verwendbaren Blocking-Proteins, beispielsweise BSA, behandelt werden. Nach dem Entfernen der Lösung können die Dünnschichtwellenleiter oder Biochips getrocknet und gelagert werden.

Das Aufbringen der Probe und der vorzugsweise fluoreszent markierten Mykotoxin-bindenden Bindungspartner zu den immobilisierten Erkennungselementen auf dem Dünnschichtwellenleiter, bevorzugt Evaneszent-Feld-Biochip, vorzugsweise einen planaren optischen Wellenleiter-Biochip, kann gleichzeitig oder nacheinander erfolgen. So kann die Zugabe von vorzugsweise fluoreszent markierten Bindungspartnern, beispielsweise von einem oder mehreren vorzugsweise fluoreszenzmarkierten Antikörpern gegen ein oder mehrere Mykotoxine, vor oder während der Inkubation einer Probe, beispielsweise eines Extrakts auf dem Chip erfolgen. Es kann auch beispielsweise ein Extrakt einer zu untersuchenden Probe im Gemisch mit bevorzugt markierten, vorzugsweise fluoreszenzmarkierten Antikörpern gegen ein oder mehrere Mykotoxine auf den Chip gegeben werden.

Von besonderem Vorteil ist, dass die Probe gemäß dem erfindungsgemäßen Verfahren weniger als 15 Minuten, vorzugsweise weniger als 10 Minuten, besonders bevorzugt weniger als 5 Minuten, vor der Detektion des Signals mit den immobilisierten Bindungspartnern als chemisches oder biochemisches Erkennungselement auf dem Dünnschichtwellenleiter und/oder den Bindungspartnern inkubieren kann.

Diese ermöglicht einen großen Vorteil gegenüber bekannten Verfahren, die Inkubationszeiten mit aufgebrachtem Mykotoxinkonjugat mit einer Lösung von markierten Mykotoxinantikörpern von teilweise bis zu zwei Stunden benötigen, oder gegenüber Verfahren, die Vorinkubationen der Proben mit markierten Anti-Mykotoxin-Antikörpern erfordern. Dies ermöglicht, dass die Bestimmung der Mykotoxine gemäß dem erfindungsgemäßen Verfahren gegenüber bekannten Verfahren deutlich schneller erfolgen kann, Insbesondere kann die Inkubationszeit deutlich verkürzt werden. In besonders bevorzugten Ausführungsformen kann die Inkubationszeit unter 10 Minuten oder sogar lediglich 5 Minuten betragen. Insbesondere in Kombination mit dem weiteren Vorteil, dass auf Waschschritte verzichtet werden kann, wird ermöglicht, dass durch das erfindungsgemäße Verfahren ein Ergebnis in weniger als 20 Minuten, vorzugsweise in weniger als 15, besonders bevorzugt in weniger als 10 Minuten vorliegen kann.

Unter Verwendung des erfindungsgemäßen Verfahrens können Mykotoxine quantitativ und vorzugsweise mit geringer Variation bestimmt werden. Beispielsweise kann der sogenannte Interlabor-Variationskoeffizient, ein Maß für die Vergleichspräzision, weniger als 50 %, bevorzugt weniger als 40 % betragen. Weiterhin kann der sogenannte Intralabor-Variationskoeffizient, ein Maß für die Wiederholpräzision, weniger als 20 % betragen. Dies ermöglicht die Verwendung des erfindungsgemäßen Verfahrens im Rahmen eines standardisierten und einfachen Verfahrens zur Bestimmung von Mykotoxinen in Nahrungsmitteln, beispielsweise Getreide, Getreideprodukten oder Wein.

Nachweisbare Mykotoxine sind vorzugsweise ausgewählt aus der Gruppe umfassend Aflatoxine, Ochratoxine, Mutterkornalkaloide, Patulin und/oder Fusarientoxine beispielsweise ausgewählt aus der Gruppe umfassend Deoxynivalenol, Nivalenol, Zearalenon, T-2 Toxin, HT-2 Toxin, Ochratoxin A und/oder Fumonisine. Fumonisine sind vorzugsweise ausgewählt aus der Gruppe umfassend Fumonisin B1, Fumonisin B2 und/oder Fumonisin B3.

Entsprechend sind verwendbare Bindungspartner bevorzugt ausgewählt aus der Gruppe der Mykotoxine umfassend Aflatoxine, Ochratoxine, Mutterkornalkaloide, Patulin und/oder Fusarientoxine beispielsweise ausgewählt aus der Gruppe umfassend Deoxynivalenol, Nivalenol, Zearalenon, T-2 Toxin, HT-2 Toxin, Ochratoxin A und/oder Fumonisine und Antikörper gegen Mykotoxine ausgewählt aus der Gruppe umfassend Aflatoxine, Ochratoxine, Mutterkornalkaloide, Patulin und/oder Fusarientoxine beispielsweise ausgewählt aus der Gruppe umfassend Deoxynivalenol, Nivalenol, Zearalenon, T-2 Toxin, HT-2 Toxin und/oder Fumonisine.

Abhängig von der Art des verwendeten Immunoassay werden einer der Bindungspartner, beispielsweise im Fall eines indirekt kompetitiven Immnunoassays eines oder mehrere der Mykotoxine, als Erkenhungselement auf dem Dünnschiehtwellenleiter immobilisiert, während der andere Bindungspartner, beispielsweise im Fall eines indirekt kompetitiven Immnunoassays einer oder mehrere der Anti-Mykotoxin-Antikörper, vor oder gleichzeitig mit der Probe, auf den Dünnschichtwellenleiter gegeben werden. hierbei ist der zuzugebende Bindungspartner vorzugsweise lumineszenzfähig markiert, bevorzugt mit einem Fluorophor markiert.

Vorzugsweise sind verwendbare Bindungspartner ausgewählt aus der Gruppe umfassend Deoxynivalenol, Nivalenol, Zearalenon, T-2 Toxin, HT-2 Toxin, Ochratoxin A und/oder Fumonisin B1, Fumonisin B2 und/oder Fumonisin B3 und Antikörper gegen Mykotoxine ausgewählt aus der Gruppe umfassend aus der Gruppe umfassend Deoxynivalenol, Nivalenol, Zearalenon, T-2 Toxin, HT-2 Toxin, Ochratoxin A und/oder Fumonisin B1, Fumonisin B2 und/oder Fumonisin B3.

Hierbei sind vorzugsweise monoklonale Antikörper gegen Mykotoxin verwendbar, beispielsweise Anti-Fumosin B1, Anti-Fumosin B2 oder Anti-Fumosin B3. Weiterhin verwendbar sind Antikörper, die gegen die Gruppe der Fumosine wirken. Verwendbare Bindungspartner, vorzugsweise Antikörper gegen Mykotoxine, können einzeln oder im Gemisch verwendet werden, weiterhin sind ebenfalls kreuzreaktive Antikörper verwendbar.

Ein besonderer Vorteil des erfindungsgemäßen Verfahrens ergibt sich daraus, dass Mykotoxine durch das erfindungsgemäße Verfahren mit erhöhter Sensitivität nachweisbar sind. Beispielsweise können Mykotoxine, insbesondere in Nahrungsmitteln für Mensch oder Tier, beispielsweise Getreide, Wein, Säfte, Früchte und/oder Produkte daraus, oder in Extrakten der Nahrungsmittel oder Produkte noch im Bereich nanomolarer oder picomolarer Konzentrationen der Mykotoxine nachweisbar sein. Beispielsweise können Mykotoxine in Getreideextrakt noch im Bereich von 0,1 pM bis 100 nM Mykotoxin, vorzugsweise im Bereich von 1 pM bis 1 nM Mykotoxin, nachgewiesen werden. insbesondere können Konzentrationen von weniger als 1 nM, vorzugsweise von weniger als 100 pM Mykotoxin, bevorzugt von weniger als 10 pM Mykotoxin, besonders bevorzugt weniger als 1 pM Mykotoxin nachweisbar sein.

Weiterhin können Mykotoxine in Getreideextrakt im Bereich von 10⁻⁴ ppb bis 10000 ppb Mykotoxin, in Getreide im Bereich von 10⁻² ppb bis 10000 ppb Mykotoxin, nachweisbar sein. Vorzugsweise können Mykotoxine in Getreideextrakt im Bereich von ≤ 0,1 ppb Mykotoxin, bevorzugt im Bereich von ≤ 0,01 ppb Mykotoxin, besonders bevorzugt im Bereich von ≤10⁻⁴ ppb Mykotoxin, in Getreide im Bereich von ≤ 0,1 ppb Mykotoxin, bevorzugt im Bereich von ≤ 0,01 ppb Mykotoxin, besonders bevorzugt im Bereich von ≤ 10⁻⁴ ppb Mykotoxin, nachweisbar sein.

Dies ermöglich außerhalb eines analytischen Labors eine genauere Bestimmung der in Nahrungsmitteln enthaltenen Mykotoxine als bislang möglich.

Das erfindungsgemäße Verfahren ermöglicht, dass man wenigstens zwei Mykotoxine, vorzugsweise 2 bis 1000 Mykotoxine, bevorzugt 5 bis 100 Mykotoxine nachweisen kann. Insbesondere können die Mykotoxine gleichzeitig bestimmt werden. Dies bedeutet einen großen Vorteil gegenüber bekannten Verfahren, die zumeist lediglich den Nachweis eines einzigen Mykotoxins gleichzeitig erlauben.

Eine bevorzugte Ausführungsform des Verfahrens zum Nachweis von Mykotoxinen sieht vor, dass man auf der Oberfläche eines Dünnschichtwellenleiters umfassend eine erste optisch transparente wellenleitende Schicht (a) auf einer zweiten optisch transparenten Schicht (b), wobei (b) einen niedrigeren Brechungsindex besitzt als (a) auf Schicht (a) Mykotoxin-Rinderserumalbumin-Konjugate als spezifische und/oder affine Bindungspartner für einen Mykotoxin-bindenden Bindungspartner räumlich separiert immobilisiert. Anschließend können die zu analysierende Probe und die vorzugsweise mit einem Fluorophor markierten Mykotoxin-bindenden Bindungspartner gleichzeitig oder aufeinanderfolgend zugegeben werden. Die spezifische und/oder affine Wechselwirkung zwischen den auf dem Dünnschichtwellenleiter immobilisierten Mykotoxin-Rinderserumalbumin-Konjugaten, den Mykotoxin(en) der Probe und/oder den vorzugsweise mit einem Fluorophor markierten Mykotoxin-bindenden Bindungspartnern kann als Signaländerung im evaneszenten Feld detektiert werden. Die Anwesenheit eines Mykotoxins in der Probe führt dabei zu einer Änderung des Signals im evaneszenten Feld.

Erfindungsgemäß kann der Nachweis der Mykotoxine durch einen Assay, beispielsweise einen Immuno-Assay auf dem Chip erfolgen. Der Nachweis der Mykotoxine erfolgt vorzugsweise in Form eines Immunoassays, bevorzugt eines kompetitiven Immunoassays, beispielsweise eines direkt oder indirekt kompetitiven Immunoassays, besonders bevorzugt in Form eines indirekt kompetitiven Immunoassays.

Ein Verfahren zum Nachweis von Mykotoxinen in Form eines direkt kompetitiven Immunoassays kann vorsehen, dass man auf der Oberfläche eines Dünnschichtwellenleiters umfassend eine erste optisch transparente wellenleitende Schicht (a) auf einer zweiten optisch transparenten Schicht (b), wobei (b) einen niedrigeren Brechungsindex besitzt als (a), Anti-Mykotoxin-Antikörper als chemisches oder biochemisches Erkennungselement für Mykotoxine räumlich separiert immobilisiert. Anschließend können vorzugsweise mit einem Fluorophor markierte Mykotoxine oder vorzugsweise mit einem Fluorophor markierte Mykotoxin-BSA-Konjugate gleichzeitig oder vor der zu analysierenden Probe zugegeben werden. Die Wechselwirkung zwischen den auf dem Dünnschichtwellenleiter immobilisierten Anti-Mykotoxin-Antikörper, den Mykotoxin(en) der Probe und/oder den vorzugsweise mit einem Fluorophor markierten Mykotoxine oder Mykotoxin-BSA-Konjugate kann als Signaländerung im evaneszenten Feld detektiert werden.

Im Falle eines direkten kompetitiven Immuno-Assays können, vorzugsweise zwei oder mehrere verschiedene, Anti-Mykotoxin-Antikörper auf der Chip-Oberfläche kovalent oder nichtkovalent immobilisiert werden, beispielsweise durch Spotting. Wird beispielsweise ein Extrakt einer zu untersuchenden Probe im Gemisch mit vorzugsweise fluoreszenzmarkierten Mykotoxinen oder Mykotoxin-Konjugaten auf den Chip gegeben, kommt es zur Kompetition der markierten und unmarkierten Mykotoxine bzw. Mykotoxin-Konjugate um die auf dem Chip verfügbaren Antikörper-Bindungsstellen. Die Zugabe der fluoreszenzmarkierten Mykotoxine kann vor oder während der Inkubation des Extrakts auf dem Chip erfolgen. Die Menge der an die immobilisierten Antikörper gebundenen markierten Mykotoxine ist umgekehrt proportional zu der im Extrakt befindlichen Menge an Mykotoxinen.

Der Nachweis kann auch in Form eines Sandwich-Assays ausgeführt werden. In diesem Fall werden anstelle der markierten Mykotoxine oder Mykotoxin-Konjugate markierte Detektionsantikörper verwendet, die an einen immobilisierten Komplex aus auf dem Chip immobilisierten Antikörper und Mykotoxin binden. Beim Sandwich-Assay ist die Menge der an die Antikörper gebundenen Fluorophore proportional zu der Konzentration der Mykotoxine im Extrakt.

Die bevorzugte Ausführungsform des Verfahrens zum Nachweis von Mykotoxinen in Form eines indirekt kompetitiven Immunoassays kann vorsehen, dass man auf der Oberfläche eines Dünnschichtwellenleiters umfassend eine erste optisch transparente wellenleitende Schicht (a) auf einer zweiten optisch transparenten Schicht (b), wobei (b) einen niedrigeren Brechungsindex besitzt als (a), vorzugsweise mit einem Fluorophor markierte Mykotoxin-BSA-Konjugate als chemisches oder biochemisches Erkennungselement räumlich separiert immobilisiert. Anschließend können vorzugsweise mit einem Fluorophor markierte Anti-Mykotoxin-Antikörper gleichzeitig oder vor der zu analysierenden Probe zugegeben werden. Die Wechselwirkung zwischen den auf dem Dünnschichtwellenleiter immobilisierten vorzugsweise mit einem Fluorophor markierte Mykotoxin-BSA-Konjugate, den Mykotoxin(en) der Probe und/oder den vorzugsweise mit einem Fluorophor markierten Anti-Mykotoxin-Antikörper kann als Signaländerung im evaneszenten Feld detektiert werden.

Erfindungsgemäß kann der Nachweis der Mykotoxine auch durch einen indirekten, kompetitiven Immuno-Assay erfolgen. In diesem Fall können Mykotoxin-BSA-Konjugate, auf dem Chip immobilisiert werden. Wird ein Extrakt einer zu untersuchenden Probe im Gemisch mit vorzugsweise fluoreszenzmarkierten Anti-Mykotoxin-Antikörpem auf den Chip gegeben, kommt es zur Kompetition der immobilisierten und der in Lösung befindlichen Mykotoxine um die verfügbaren Bindungsstellen der fluoreszenzmarkierten Antikörper. Die Zugabe der fluoreszenzmarkierten Anti-Mykotoxin-Antikörper kann vor oder während der Inkubation des Extrakts auf dem Chip erfolgen. Die Menge der gebundenen markierten Antikörper ist in diesem Fall umgekehrt proportional zu der im Extrakt befindlichen Menge an Mykotoxinen.

Weiterhin ist von Vorteil, dass die Detektion des Signals im Evaneszentfeld mittels eines Auslesegeräts erfolgen kann. Das Auslesegerät kann beispielsweise ein robustes und kostengünstiges Auslesegerät sein.

Die Auswertung der Signalintensität, beispielsweise der Fluoreszenzintensität kann mittels einer geeigneten Software erfolgen, ebenso wie die Berechnung der in der Probe vorliegenden Menge der Mykotoxine.

Die zur Verfügung gestellten Vorteile des erfindungsgemäßen Verfahrens, insbesondere eine Kombination eines einfach durchführbaren Verfahrens, der Möglichkeit gleichzeitig mehrere Mykotoxine quantitativ auf einem robusten und kostengünstigen Auslesegerät nachweisen zu können, ermöglicht einen einfachen und schnellen Nachweis von Mykotoxinen außerhalb eines analytischen Labors.

Eine Vorrichtung zur Durchführung des Verfahrens zum Nachweis von Mykotoxinen weist einen Dünnschichtwellenleiter, bevorzugt einen planaren optischen Wellenleiter-Biochip auf, der auf einem Dünnschichtwellenleiter basiert, umfassend eine erste optisch transparente wellenleitende Schicht (a) auf einer zweiten optisch transparenten Schicht (b), wobei (b) einen niedrigeren Brechungsindex besitzt als (a). Vorzugsweise sind die Erkennungselemente auf der Schicht (a) immobilisiert.

Geeignete planare optische Wellenleiter sind beilspielsweise in der WO 01/92870 oder in US 5,959,292 beschrieben.

In bevorzugten Ausführungsformen der Vorrichtung kann die optisch transparente Schicht (b) des Dünnschichtwellenleiters, vorzugsweise planaren optischen Wellenleiter-Biochips, aus Silikaten wie Glas oder Quarz oder aus einem transparenten Kunststoff, bevorzugt ausgewählt aus der Gruppe umfassend Polycarbonate, Polyimide, Polymethacrylate, Polystyrene, zyklische Polyolefine, und/oder zyklische Polyolefin-Copolymere vorzugsweise aus zyklischen Polyolefinen oder zyklischen Polyolefin-Copolymeren, ausgebildet sein. Geeignete Kunststoffe zur Herstellung der optisch transparenten Schicht (b) sind beispielsweise in der WO 03/020488 beschrieben.

Bevorzugt sind transparente thermoplastische oder spritzbare Kunststoffe, beispielsweise ausgewählt aus der Gruppe umfassend Polycarbonat, Polyimid, Acrylat, insbesondere Polymethylmethacrylat, oder Polystyrol.

In bevorzugten Ausführungsformen der Vorrichtung kann die optisch transparente wellenleitende Schicht (a) Oxide ausgewählt aus der Gruppe umfassend TiO₂, ZnO, Nb₂O₅, Ta₂O₅, HfO₂ und/oder ZrO₂, bevorzugt ausgewählt aus der Gruppe umfassend TiO₂, Ta₂O₅ und/oder Nb₂O₅ umfassen. Es sind auch Kombinationen mehrerer derartiger Oxide verwendbar. Vorzugsweise ist eine optisch transparente wellenleitende Schicht (a) aus TiO₂, ZnO, Nb₂O₅, Ta₂O₅, HfO₂ oder ZrO₂, bevorzugt TiO₂, Ta₂O₅ oder Nb₂O₅ ausgebildet. Insbesondere hat sich die Verwendung von Tantalpentoxid als vorteilhaft herausgestellt.

In bevorzugten Ausführungsformen umfasst der Dünnschichtwellenleiter, insbesondere auf der optisch transparenten Schicht enthaltend Oxide ausgewählt aus der Gruppe umfassend TiO₂, ZnO, Nb₂O₅, Ta₂O₅, HfO₂ und/oder ZrO₂, Mono- oder Mehrfachschichten von Organophosphorsäuren gemäß der nachstehenden Formel (I)

R-OPO₃H₂ (I)

und/oder Organophosphonsäuren gemäß der nachstehenden Formel (II)

R-PO₃H₂ (II)

und/oder deren Salze, wobei
- R: steht für Alkyl mit C₁₀ bis C₂₄.

Bevorzugt verwendbar sind Organophosphorsäuren und/oder Organophosphonsäuren, vorzugsweise Organophosphate und/oder Organophosphonate mit R ist ausgewählt aus der Gruppe umfassend unverzweigtes Alkyl mit C₁₀ bis C₂₀, vorzugsweise ausgewählt aus der Gruppe umfassend unverzweigtes Alkyl mit C₁₂ bis C₁₈, bevorzugt Dodecylphsophorsäure, Dodecylphosphat, Octadecylphosphonat und/oder Octadecylphosphonsäure.

Vorzugsweise sind Organophosphorsäuren bzw. Organophosphate, die in Form wasserlöslicher Salze aus wässriger Lösung auf den Dünnschichtwellenleiter aufgebracht werden können.

In bevorzugten Ausführungsformen sind die Organophosphorsäuren und/oder Organophosphonsäuren, vorzugsweise Organophosphate, in Form einer Monoschicht auf den Dünnschichtwellenleiter, insbesondere einen Evaneszent-Feld-Biochip, vorzugsweise einen planaren optischen Wellenleiter-Biochip, aufgebracht.

Die Monoschicht oder Monolayer kann als eine Haftvermittlungsschicht auf die optisch transparente Schicht ausgebildet aus Oxiden aufgebracht werden. Vorteilhafter Weise können Organophosphorsäuren und/oder Organophosphonsäuren mit Erkennungselementen, insbesondere mit an Trägerproteine gekoppelten Erkennungselementen wechselwirken und die Bindung der Erkennungselemente an den Biochip verstärken.

In einer bevorzugten Form der Vorrichtung sind die Organophosphorsäuren und/oder Organophosphonsäuren, vorzugsweise Organophosphate, auf dem Dünnschichtwellenleiter vorzugsweise auf der optisch transparenten Schicht ausgebildet aus Oxiden in Form einer Haftvermittlungsschicht aufgebracht. Die Haftvermittlungsschicht kann die Bindung der Erkennungselemente an den Dünnschichtwellenleiter oder Biochip verstärken.

Es ist bevorzugt, dass die Haftvertnittlungsschicht eine Dicke von weniger als 200 nm, vorzugsweise von weniger als 20 nm, aufweist.

Vorzugsweise erfolgt die Einkopplung des Anregungslichtes in die optisch transparente wellenleitende Schicht (a) unter Verwendung von einer oder mehreren Gitterstrukturen.

Vorzugsweise ist die Gitterstruktur ein Reliefgitter mit beliebigem Profil, beispielsweise mit Rechteck-, Dreieck- oder halbkreisförmigem Profil, oder ein Phasen- oder Volumengitter mit einer periodischen Modulation des Brechungsindex in der im wesentlichen planaren optisch transparenten Schicht (a). Die Gitterstruktur kann auch ein diffraktives Gitter mit einer einheitlichen Periode sein oder ein multidiffraktives Gitter. Die Gitterstruktur kann eine senkrecht oder parallel zur Ausbreitungsrichtung des in die optisch transparente wellenleitende Schicht (a) eingekoppelten Anregungslichts räumlich variierendePeriodiziät aufweisen

Es ist bevorzugt, dass die zur Einkopplung des Anregungslichtes verwendbaren Gitterstrukturen eine Periode im Bereich von 200 nm bis 1000 nm, vorzugsweise im Bereich von 200 nm bis 400 nm aufweisen. Es ist weiterhin bevorzugt, dass die Modulationstiefe des Gitters im Bereich von 3 nm bis 60 nm, vorzugsweise im Bereich von 10 nm bis 40 nm liegt. Es wird bevorzugt, dass das Verhältnis von Modulationstiefe zur Dicke der ersten optisch transparenten wellenleitenden Schicht (a) gleich oder kleiner als 0,4 ist. Es ist ebenfalls bevorzugt, dass die Brechungsindexmodulation sowohl an der Grenzfläche zwischen Schicht a und Schicht b als auch an der Grenzfläche von Schicht a zum Analysenmedium ausgeprägt ist.

Vorzugsweise weist die optisch transparente wellenleitende Schicht (a) eine Dicke im Bereich von 40 nm bis 1000 nm, bevorzugt im Bereich von 40 nm bis 300 nm, noch bevorzugter im Bereich von 80 nm bis 200 nm, auf.

Der Unterschied in den Brechungsindizes zwischen Schicht (a) und (b) beträgt vorzugsweise ≥ 0,2 bevorzugt ≥ 0,5, und liegt noch bevorzugter bei 0,56.

Die Wellenlänge des Anregungslichts liegt vorzugsweise im Bereich von 300 nm bis 1100 nm, bevorzugt im Bereich von 300 nm bis 800 nm, noch bevorzugter im Bereich von 500 nm bis 700 nm.

Geeignetes Anregungslicht kann über eine Gitterstruktur eingekoppelt werden, an welche sich in Ausbreitungsrichtung des eingekoppelten und in der Schicht (a) geführten Lichts ein unmodulierter Bereich der Schicht (a) mit einer darauf befindlichen Vielzahl von Messbereichen in einem Array anschließt, auf denen der Nachweis der verschiedenen Mykotoxine erfolgt. Daran kann sich in der Ausbreitungsrichtung des geführten Lichts vorteilhaft eine oder mehrere weitere Gitterstrukturen mit einem dahinter befindlichen weiteren Array von Messbereichen anschließen. Alternativ können sich die Messbereiche eines Arrays oder auch einer Vielzahl von Arrays auf dem modulierten Bereich der Schicht (a) befinden.

Es wird bevorzugt, dass jedem in Ausbreitungsrichtung des eingekoppelten Anregungslichts nachfolgenden Array von Messbereichen eine für dieses Array spezifische Gitterstruktur zur Auskopplung dieses Anregungslichts zugeordnet ist, wobei senkrecht zur Ausbreitungsrichtung des eingekoppelten Anregungslichts die Gitterstrukturen spezifisch für einzelne Arrays ausgebildet sein können oder sich auch über den gesamten Dünnschichtwellenleiter in dieser Richtung erstrecken können.

Die Vorrichtung kann eine sehr große Anzahl einzelner Messfelder aufweisen. In bevorzugten Ausführungsformen der Vorrichtung sind die spezifischen und/oder affinen Bindungspartner als chemisches oder biochemisches Erkennungselement in Form von bis zu 100.000 Messfeldem oder "Spots" in einer zweidimensionalen Anordnung aufgebracht, wobei ein Messfeld oder Spot bevorzugt eine Fläche im Bereich von 0,001 mm² bis 6 mm², vorzugsweise im Bereich von 0,1 mm² bis 1 mm² aufweist. Bevorzugt sind mehr als 10, bevorzugt mehr als 50 Messfelder pro Quadratzentimeter auf dem Dünnschichtwellenleiter oder Biochip aufgebracht.

Ein weiterer Gegenstand der Erfindung ist ein Kit zum Nachweis von Mykotoxinen. Der Kit enthält wenigstens einen Dünnschichtwellenleiter umfassend eine erste optisch transparente wellenleitende Schicht (a) auf einer zweiten optisch transparenten Schicht (b), wobei (b) einen niedrigeren Brechungsindex besitzt als (a), und wobei auf der wellenleitenden Schicht (a) Mykotoxin-Rinderserumalbumin-Konjugate als chemisches oder biochemisches Erkennungselement für einen Mykotoxin-bindenden Bindungspartner räumlich separiert immobilisiert sind.

Der Kit kann weiterhin wenigstens ein Reagenz umfassend vorzugsweise fluoreszenz markierte Mykotoxin-bindende Bindungspartner enthalten. Der Kit kann auch mehrere Reagenzien umfassend vorzugsweise markierte Bindungspartner enthalten oder ein Reagenz enthaltend ein Gemisch verschiedener markierte Bindungspartner. Der Kit kann weiterhin Puffer und/oder Lösemittel enthalten, die zur Durchführung des Nachweises nach einem der vorherigen Ansprüche benötigt werden. Es kann auch vorgesehen sein, dass der Kit eine Detektionseinheit umfasst.

Die Kits sind zum Schnell-Nachweis von Mykotoxinen verwendbar.

Ein Beispiel, das der Veranschaulichung der vorliegenden Erfindung dient, ist nachstehend angegeben.

### Beispiel 1

### Ermittlung einer Standardkurve zur Messung von Zearalenon in einem indirekt kompetitiven Immuno-Assay auf einem Evaneszent-Feld-Biochip

Sieben Biochips (Firma Unaxis, Liechtenstein) mit den Außenmaßen 1 cm x 2 cm aus Glas, in das ein optisches Gitter mit einer Gittertiefe von 18 nm eingeschrieben war, versehen mit einer Schicht von 155 nm von Tantalpentoxid, wurden mit Octadecylphosphonsäure durch eintauchen in eine 500 µM Lösung von Octadecylphosphonsäure in n-Heptan/Isopropanol im Verhältnis 9:1 beschichtet. Mit Hilfe eines Spotters des Typs "Biochip Arrayer" (Perkin Elmer, Deutschland) wurden Konjugate aus Zearalenon und Bovinem Serum Albumin (ZEA-BSA, Verhältnis ZEA:BSA = 50:1, hergestellt von der Fa. Biopure, Tulln, Österreich) sowie mit dem Farbstoff DyLight 647 (Pierce, Deutschland) markierte BSA-Moleküle (DyLight 647-BSA) auf den Biochip aufgebracht. Die Spotting-Lösungen enthielten DyLight 647-BSA in einer Konzentration von 5x10⁻⁴ mg/ml in PBS (137 mM NaCl, 2,8 mM KCl, 10 mM Na₂HPO₄, 1,8 mM KH₂PO₄, pH 7,4) mit 0,1% BSA und 0,1% Tween 20, BSA-ZEA-Konjugat der Konzentration 0,5 mg/ml in PBS mit 0,1% BSA und 0,1% Tween 20. Die Spots wurden in Form neuen alternierender Reihen von jeweils 10 DyLight 647-BSA-Spots und BSA-ZEA-Konjugat-Spots in Form zweier Felder (Arrays) auf den Chip aufgebracht.

Die Spots wurden über Nacht bei hoher Luftfeuchte (40%) inkubiert, anschließend behandelte man die Biochips für 4 Stunden mit einer 3%igen Lösung von BSA in PBS. Auf die Chips wurden Meßkammern aufgebracht, sodass auf jedem Chip zwei Arrays mit getrennten Reaktionskammern gebildet wurden. Man bereitete wässrige Lösungen von Zearalenon in verschiedenen Konzentrationen im Bereich von 0 µg/l bis 31 µg/l und versetzte diese mit einem monoklonalen Anti-Zearalenon-Antikörper (Biotez, Berlin), der mit DyLight 647 markiert war, sodass man jeweils eine 1 nM Lösung des Antikörpers erhielt.

Die Mischungen unterschiedlicher Konzentrationen wurden jeweils in die Messkammern gegeben und die Biochips wurden ohne weitere Behandlungsschritte innerhalb von maximal zehn Minuten auf einem Fluoreszenz-Auslesegerät "Minifluo IV" (Bayer Technology Services, Deutschland) vermessen. Die erhaltenen Fluorezenzintensitäten für jeden Zearalenon-Spot wurden durch den Mittelwert der Fluoreszenzintensitäten des oberhalb und unterhalb des jeweiligen Spots gelegenen DyLight 647-BSA-Spots dividiert. Die Mittelwerte der Fluoreszenzintensitäten aller 40 Spots eines Arrays wurden ermittelt. Die erhaltenen konzentrationsabhängigen Fluoreszenzintensitäten wurden mit Hilfe des Computerprogramms Origin 7G (Origin Lab Corporation, USA) durch einen sigmoidalen Fit angepasst.

Es konnte festgestellt werden, dass in einem Bereich von 0,4 ppb bis 4 ppb Zearalenon, entsprechend 80% bzw. 20% der maximalen Fluoreszenzintensität in der angepaßten sigmoidalen Kurve, entsprechend einem Bereich von 1 nM bis 10 nM der eingesetzten ZEA-Konzentration in der Lösung eine Quantifizierung der ZEA-Konzentration durch Auswertung der Fluoreszenzintensität der Proben ermöglicht wurde.

### Beispiel 2

### Ermittlung einer Standardkurve zur Messung von Deoxynivalenol (DON) und Messung einer kontaminierten Futtergetreide-Probe

15 Biochips (Firma Unaxis, Liechtenstein) mit den Außenmaßen 1 cm x 2 cm aus Glas, in das ein optisches Gitter (Gittertiefe 18 nm) eingeschrieben war, versehen mit einer Schicht (155 nm) von Tantalpentoxid, wurden mit Octadecylphosphonsäure (durch Eintauchen in eine Lösung von Octadecylphosphonsäure in n-Heptan/Isopropanol 9:1) beschichtet. Mit Hilfe eines Spotters des Typs Nanoplotter (GeSiM, Deutschland) wurden Konjugate aus Deoxynivalenol und Bovinem Serum Albumin (DON-BSA, Verhältnis DON:BSA = 100:1, hergestellt von der Fa. Biopure, Tulln, Österreich) sowie Hunde-IgG (dog-IgG, Rockland, USA) auf den Biochip aufgebracht. Die Spotting-Lösungen bestanden aus Hunde-IgG in einer Konzentration von 0,2 mg/ml in PBS (137 mM NaCl, 2,8 mM KCI, 10 mM Na2HP04, 1,8 mM KH2P04, pH 7,4) mit Trehalose sowie aus BSA-DON-Konjugat der Konzentration 1 mg/ml in PBS mit Trehalose. Die Spots wurden in Form zweier Reihen von jeweils 12 Hunde-IgG-Spots und einer dazwischen liegenden Reihe von 12 BSA-DON-Konjugat-Spots in Form zweier Felder (Arrays) auf den Chip aufgebracht.

Die Spots wurden 1h bei 37°C inkubiert, anschließend behandelte man die Biochips für bis zu 4 Stunden mit einer Lösung von BSA in PBS. Auf die Chips wurden Meßkammern aufgebracht, sodass auf jedem Chip zwei Arrays mit getrennten Reaktionskammern gebildet wurden. Man extrahierte 5g nichtkontaminiertes Weizenmehl mit einer Lösung von 70% Methanol in Wasser (v/v) durch Schütteln für 5 min. Der Extrakt wurde zentrifugiert und anschließend mit einem Tris-CitratPuffer (pH 7,4), der BSA, Casein, fettarmes Trockenmilchpulver, Tween 20, Polyethylenglycol und Sucrose enthielt, im Verhältnis 1:4 v/v (Extrakt:Puffer) verdünnt. Man bereitete Lösungen von Deoxynivalenol in verschiedenen Konzentrationen (15 bis 150 µg/l) und versetzte diese mit einem monoklonalen Anti-Deoxynivalenol-Antikörper, der mit DyLight 647 markiert war sowie mit einem monoklonalen Ziege-Anti-Hunde-IgG Antikörper, der ebenfalls mit DyLight 647 markiert war, sodass man jeweils eine 1nM Lösung des Antikörpers erhielt.

Die Lösungen unterschiedlicher Konzentrationen wurden jeweils in die Meßkammern gegeben und die Biochips wurden ohne weitere Behandlungsschritte innerhalb von maximal zehn Minuten auf einem Fluoreszenz-Auslesegerät "Minifluo IV" (Bayer Technology Services, Deutschland) vermessen. Die erhaltenen Fluorezenzintensitäten für jeden Deoxynivalenol-Spot wurden durch den Mittelwert der Fluoreszenzintensitäten des oberhalb und unterhalb des jeweiligen Spots gelegenen Hunde-IgG-Spots dividiert. Die normierten Mittelwerte der Fluoreszenzintensitäten aller 12 DON-Spots eines Arrays wurden ermittelt. Die erhaltenen konzentrationsabhängigen, normierten Fluoreszenzintensitäten wurden mit Hilfe eines Computerprogramms durch einen potentiellen Fit angepasst.

Analog zu dem oben dargestellten Extraktionsverfahren wurden 5g einer mit DON kontaminierten Futtergetreidemehl-Probe (Fa Coring, Deutschland, zertifiziert 526 ppb DON) extrahiert und der Extrakt verdünnt. 300 µl des verdünnten Extraktes wurden mit einem monoklonalen Anti-Deoxynivalenol-Antikörper, der mit DyLight 647 markiert war sowie mit einem monoklonalen Ziege-Anti-Hunde-IgG Antikörper, der ebenfalls mit DyLight647 markiert war versetzt, sodass man jeweils eine 1nM Lösung des Antikörpers erhielt. Jeweils 100 µl der Lösung wurden in eine Messkammer gegeben und die Biochips wurden ohne weitere Behandlungsschritte innerhalb von maximal zehn Minuten auf einem Fluoreszenz-Auslesegerät "Minifluo IV" (Bayer Technology Services, Deutschland) vermessen. Die erhaltenen Fluorezenzintensitäten für jeden Deoxynivalenol-Spot wurden durch den Mittelwert der Fluoreszenzintensitäten des oberhalb und unterhalb des jeweiligen Spots gelegenen Hunde-IgG-Spots dividiert. Die normierten Mittelwerte der Fluoreszenzintensitäten aller 12 DON-Spots eines Arrays wurden ermittelt. Die erhaltenen Fluoreszenzintensitäten wurden mithilfe der oben beschriebenen Standardkurve in DON-Konzentrationen im Futtergetreidemehl umgerechnet, wobei ein durchschnittlicher Wert über die drei Messungen von 590 ppb erhalten wurde.

## Patentansprüche

1. Verfahren zum Schnell-Nachweis von Mykotoxinen, umfassend die folgenden Schritte:
a) Bereitstellen eines Dünnschichtwellenleiters umfassend eine erste optisch transparente wellenleitende Schicht (a) auf einer zweiten optisch transparenten Schicht (b), wobei (b) einen niedrigeren Brechungsindex besitzt als (a), und wobei auf Schicht (a) Mykotoxin- Rinderserumalbumin - Konjugate als chemisches oder biochemisches Erkennungselement für einen Mykotoxin-bindenden Bindungspartner räumlich separiert immobilisiert werden,
b) Aufbringen einer Mykotoxin(e) enthaltenden Probe und von Mykotoxin-bindenden Bindungspartnern zu den immobilisierten Mykotoxin-Rinderserumalbumin -Konjugate auf dem Dünnschichtwellenleiter, wobei ein Markierungselement an einen Bindungspartner oder an den immobilisierten Mykotoxin- Rinderserumalbumin -Konjugate gebunden ist,
c) Detektieren eines Signals im Evaneszentfeld, das durch Änderung der optischen Eigenschaften an der Grenzfläche zum Wellenleiter infolge der Wechselwirkung der auf dem Dünnschichtwellenleiter immobilisierten Mykotoxin- Rinderserumalbumin -Konjugate mit den Mykotoxin-bindenden Bindungspartnern hervorgerufen wird,
dann
d) Bestimmen der in der Probe vorliegenden Menge an Mykotoxin(en).

2. Verfahren nach Anspruch 1, **dadurch gekennzeichnet, dass** man auf Schicht (a) Mono- oder Mehrfachschichten von Organophosphorsäuren gemäß der nachstehenden Formel (I)
R-OPO₃H₂ (I)
und/oder Organophosphonsäuren gemäß der nachstehenden Formel (II)
R-PO₃H₂ (II)
und/oder deren Salze aufbringt, wobei
R steht für Alkyl mit C₁₀ bis C₂₄.

3. Verfahren nach Anspruch 2, **dadurch gekennzeichnet, dass** man Organophosphorsäuren, Organophosphonsäuren, Organophosphate und/oder Organophosphonate mit R ausgewählt aus der Gruppe umfassend unverzweigtes Alkyl mit C₁₀ bis C₂₀, vorzugsweise ausgewählt aus der Gruppe umfassend unverzweigtes Alkyl mit C₁₂ bis C₁₈, bevorzugt Organophosphorsäuren, Organophosphonsäuren, Organophosphate und/oder Organophosphonate ausgewählt aus der Gruppe umfassend Dodecylphsophorsäure, Dodecylphosphat, Octadecylphosphonat und/oder Octadecylphosphonsäure, verwendet.

4. Verfahren nach einem der vorherigen Ansprüche, **dadurch gekennzeichnet, dass** man einen Dünnschichtwellenleiter umfassend eine optisch transparente wellenleitende Schicht (a) umfassend Oxide ausgewählt aus der Gruppe umfassend TiO₂, ZnO, Nb₂O₅, Ta₂O₅, HfO₂ und/oder ZrO₂, bevorzugt ausgewählt aus der Gruppe umfassend TiO₂. Ta₂O₅ und/oder Nb₂O₅ verwendet.

5. Verfahren nach einem der vorherigen Ansprüche, **dadurch gekennzeichnet, dass** man die Mykotoxin-bindenden Bindungspartner auswählt aus der Gruppe umfassend Anti-Mykotoxin-Antikörper, Anti-Mykotoxin-Antikörper-Konjugate, Fragmente von Anti-Mykotoxin-Antikörpern, Mykotoxin-bindende Peptide, Mykotoxin-bindende Anticaline, Mykotoxin-bindende Aptamere, Mykotoxin-bindende Spiegelmere und/oder Mykotoxin-bindende imprinted Polymers, vorzugsweise auswählt aus der Gruppe umfassend Anti-Mykotoxin-Antikörper, Anti-Mykotoxirt-Antikörper-Konjugate.

6. Verfahren nach einem der vorherigen Ansprüche, **dadurch gekennzeichnet, dass** man das Markierungselement, vorzugsweise ein Fluorophor, mittels eines Proteins, vorzugsweise mittels Rinderserumalbumin an Mykotoxine bindet.

7. Verfahren nach einem der vorherigen Ansprüche, **dadurch gekennzeichnet, dass** die Probe ein Nahrungsmittel für Mensch oder Tier, bevorzugt ausgewählt aus der Gruppe umfassend Getreide, Wein, Säfte, Früchte und/oder Getreide, Wein, Säfte und/oder Früchte enthaltende Produkte, oder ein mit einem Lösemittel oder Lösemittelgemisch extrahierter Extrakt der Nahrungsmittel oder Produkte ist.

8. Verfahren nach einem der vorherigen Ansprüche, **dadurch gekennzeichnet, dass** man die Probe weniger als 15 Minuten, vorzugsweise weniger als 10 Minuten vor der Detektion des Signals mit den Mykotoxin-Rinderserumalbumin-Konjugaten als chemisches oder biochemisches Erkennungselement und/oder den Mykotoxin-bindenden Bindungspartnern inkubiert.

9. Verfahren nach einem der vorherigen Ansprüche, **dadurch gekennzeichnet, dass** die Mykotoxine ausgewählt sind aus der Gruppe umfassend Aflatoxine, Ochratoxine, Mutterkornalkaloide, Patulin und/oder Fusarientoxine beispielsweise ausgewählt aus der Gruppe umfassend Deoxynivalenol, Nivalenol, Zearalenon, T-2 Toxin, HT-2 Toxin und/oder Fumonisine, vorzugsweise ausgewählt aus der Gruppe umfassend Ochratoxin A, Deoxynivalenol, Nivalenol, Zearalenon, T-2 Toxin, HT-2 Toxin, Fumonisin B1, Fumonisin B2 und/oder Fumonisin B3.

10. Verfahren nach einem der vorherigen Ansprüche, **dadurch gekennzeichnet, dass** man den Nachweis in Form eines Immunoassays führt.

11. Kit zum Schnell-Nachweis von Mykotoxinen, **dadurch gekennzeichnet, dass** der Kit wenigstens einen Dünnschichtwellenleiter umfassend eine erste optisch transparente wellenleitende Schicht (a) auf einer zweiten optisch transparenten Schicht (b), wobei (b) einen niedrigeren Brechungsindex besitzt als (a), und wobei auf der wellenleitende Schicht (a) Mykotoxin- Rinderserumalbumin -Konjugate als chemisches oder biochemisches Erkennungselement für einen Mykotoxin-bindenden Bindungspartner räumlich separiert immobilisiert sind, enthält.

12. Kit nach Anspruch 11, **dadurch gekennzeichnet, dass** das Kit ein Reagenz umfassend vorzugsweise fluoreszenzmarkierte Mykotoxin-bindenden Bindungspartnern enthält.

13. Verwendung eines Kits nach einem der vorherigen Ansprüche zum Schnell-Nachweis von Mykotoxinen.

## Claims

1. Process for rapid detection of mycotoxins, comprising the following steps:
a) providing a thin-film waveguide comprising a first optically transparent wave-guiding layer (a) on top of a second optically transparent layer (b), with (b) having a lower refractive index than (a), and with mycotoxin-bovine serum albumin conjugates being immobilized on layer (a) as a chemical or biochemical recognition element for a mycotoxin-binding binding partner in a spatially separated manner,
b) applying a mycotoxin(s)-containing sample and mycotoxin-binding binding partners to the immobilized mycotoxin-bovine serum albumin conjugates on said thin-film waveguide, with a labeling element being bound to a binding partner or to the immobilized mycotoxin-bovine serum albumin conjugates,
c) detecting a signal in the evanescent field which is caused by a change in the optical properties at the interface to the waveguide as a result of the interaction of the mycotoxin-bovine serum albumin conjugates immobilized on the thin-film waveguide with the mycotoxin-binding binding partners,
then
d) determining the amount of mycotoxin(s) present in the sample.

2. Process according to Claim 1, **characterized in that** mono- or multilayers of organophosphoric acids of the following formula (I)
R-OPO₃H₂ (I)
and/or organophosphonic acids of the following formula (II)
R-PO₃H₂ (II)
and/or their salts are applied to layer (a), where
R is a C₁₀ to C₂₄ alkyl.

3. Process according to Claim 2, **characterized in that** organophosphoric acids, organophosphonic acids, organophosphates and/or organophosphonates, with R being selected from the group comprising unbranched C₁₀ to C₂₀ alkyl, preferably selected from the group comprising unbranched C₁₂ to C₁₈ alkyl, preferably organophosphoric acids, organophosphonic acids, organophosphates and/or organophosphonates selected from the group comprising dodecylphsophoric acid, dodecylphosphate, octadecylphosphonate and/or octadecylphosphonic acid, are used.

4. Process according to any of the preceding claims, **characterized in that** a thin-film waveguide comprising an optically transparent wave-guiding layer (a) comprising oxides selected from the group comprising TiO₂, ZnO, Nb₂O₅, Ta₂O₅, HfO₂ and/or ZrO₂, preferably selected from the group comprising TiO₂, Ta₂O₅ and/or Nb₂O₅, is used.

5. Process according to any of the preceding claims, **characterized in that** the mycotoxin-binding binding partners are selected from the group comprising anti-mycotoxin antibodies, anti-mycotoxin-antibody conjugates, fragments of anti-mycotoxin antibodies, mycotoxin-binding peptides, mycotoxin-binding anticalins, mycotoxin-binding aptamers, mycotoxin-binding spiegelmers and/or mycotoxin-binding imprinted polymers, preferably selected from the group comprising anti-mycotoxin antibodies, anti-mycotoxin-antibody conjugates.

6. Process according to any of the preceding claims, **characterized in that** the labeling element, preferably a fluorophore, is bound to mycotoxins by means of a protein, preferably by means of bovine serum albumin.

7. Process according to any of the preceding claims, **characterized in that** the sample is a food item for humans or animals, preferably selected from the group comprising cereals, wine, juices, fruits and/or products containing cereals, wine, juices and/or fruits, or an extract of said food items or products which has been extracted with a solvent or solvent mixture.

8. Process according to any of the preceding claims, **characterized in that** the sample is incubated with the mycotoxin-bovine serum albumin conjugates as chemical or biochemical recognition element and/or the mycotoxin-binding binding partners less than 15 minutes, preferably less than 10 minutes, before detection of the signal.

9. Process according to any of the preceding claims, **characterized in that** the mycotoxins are selected from the group comprising aflatoxins, ochratoxins, ergot alkaloids, patulin and/or fusarium toxins, for example selected from the group comprising deoxynivalenol, nivalenol, zearalenone, T-2 toxin, HT-2 toxin and/or fumonisins, preferably selected from the group comprising ochratoxin A, deoxynivalenol, nivalenol, zearalenone, T-2 toxin, HT-2 toxin, fumonisin B1, fumonisin B2 and/or fumonisin B3.

10. Process according to any of the preceding claims, **characterized in that** detection is carried out by way of an immunoassay.

11. Kit for rapid detection of mycotoxins, **characterized in that** the kit comprises at least one thin-film waveguide comprising a first optically transparent wave-guiding layer (a) on top of a second optically transparent layer (b), with (b) having a lower refractive index than (a), and with mycotoxin-bovine serum albumin conjugates being immobilized on the wave-guiding layer (a) as a chemical or biochemical recognition element for a mycotoxin-binding binding partner in a spatially separated manner.

12. Kit according to Claim 11, **characterized in that** it comprises a reagent preferably comprising fluorescently labeled mycotoxin-binding binding partners.

13. Use of a kit according to any of the preceding claims for rapid detection of mycotoxins.

## Revendications

1. Procédé de détection rapide de mycotoxines, comprenant les étapes suivantes :
a) la préparation d'un guide d'ondes à couches minces comprenant une première couche guide d'ondes optiquement transparente (a) sur une seconde couche optiquement transparente (b), (b) présentant un indice de réfraction inférieur à (a) et des conjugués mycotoxine-albumine de sérum bovin étant immobilisés de manière séparée dans l'espace sur la couche (a) en tant qu'élément de reconnaissance chimique ou biochimique pour un partenaire de liaison se liant aux mycotoxines,
b) l'application d'un échantillon contenant une ou plusieurs mycotoxines et de partenaires de liaison se liant aux mycotoxines sur les conjugués mycotoxine-albumine de sérum bovin immobilisés sur le guide d'ondes à couches minces, un élément de marquage étant relié à un partenaire de liaison ou aux conjugués mycotoxine-albumine de sérum bovin immobilisés,
c) la détection d'un signal dans le champ évanescent qui est produit par une modification des propriétés optiques au niveau de l'interface du guide d'ondes en conséquence de l'interaction des conjugués mycotoxine-albumine de sérum bovin immobilisés sur le guide d'ondes à couches minces avec les partenaires de liaison se liant aux mycotoxines,
puis
d) la détermination de la quantité de la ou des mycotoxines présentes dans l'échantillon.

2. Procédé selon la revendication 1, **caractérisé en ce que** des mono- ou multicouches d'acides organophosphoriques selon la formule (I) ci-dessous
R-OPO₃H₂ (I)
et/ou d'acides organophosphoniques selon la formule (II) ci-dessous
R-PO₃H₂ (II)
et/ou de leurs sels sont appliquées sur la couche (a),
R représentant un alkyle en C₁₀ à C₂₄.

3. Procédé selon la revendication 2, **caractérisé en ce que** des acides organophosphoriques, des acides organophosphoniques, des organophosphates et/ou des organophosphonates avec R choisi dans le groupe comprenant les alkyles non ramifiés en C₁₀ à C₂₀, de préférence choisi dans le groupe comprenant les alkyles non ramifiés en C₁₂ à C₁₈, de préférence des acides organophosphoriques, des acides organophosphoniques, des organophosphates et/ou des organophosphonates choisis dans le groupe comprenant l'acide dodécylphosphorique, le phosphate de dodécyle, le phosphonate d'octadécyle et/ou l'acide octadécylphosphonique, sont utilisés.

4. Procédé selon l'une quelconque des revendications précédentes, **caractérisé en ce qu'**un guide d'ondes à couches minces comprenant une couche guide d'ondes optiquement transparente (a) comprenant des oxydes choisis dans le groupe comprenant TiO₂, ZnO, Nb₂O₅, Ta₂O₅, HfO₂ et/ou ZrO₂, de préférence choisis dans le groupe comprenant TiO₂, Ta₂O₅ et/ou Nb₂O₅, est utilisé.

5. Procédé selon l'une quelconque des revendications précédentes, **caractérisé en ce que** les partenaires de liaison se liant aux mycotoxines sont choisis dans le groupe comprenant les anticorps anti-mycotoxines, les conjugués d'anticorps anti-mycotoxines, les fragments d'anticorps anti-mycotoxines, les peptides se liant aux mycotoxines, les anticalines se liant aux mycotoxines, les aptamères se liant aux mycotoxines, les spiegelmères se liant aux mycotoxines et/ou les polymères imprimés se liant aux mycotoxines, de préférence choisis dans le groupe comprenant les anticorps anti-mycotoxines, les conjugués d'anticorps anti-mycotoxines.

6. Procédé selon l'une quelconque des revendications précédentes, **caractérisé en ce que** l'élément de marquage, de préférence un fluorophore, est relié aux mycotoxines par une protéine, de préférence par une albumine de sérum bovin.

7. Procédé selon l'une quelconque des revendications précédentes, **caractérisé en ce que** l'échantillon est un aliment pour les hommes ou les animaux, de préférence choisi dans le groupe comprenant les céréales, le vin, les jus, les fruits et/ou les produits contenant des céréales, du vin, des jus et/ou des fruits, ou un extrait des aliments ou produits extrait avec un solvant ou un mélange de solvants.

8. Procédé selon l'une quelconque des revendications précédentes, **caractérisé en ce que** l'échantillon est incubé pendant moins de 15 minutes, de préférence moins de 10 minutes, avec le conjugué mycotoxine-albumine de sérum bovin en tant qu'élément de reconnaissance chimique ou biochimique et/ou les partenaires de liaison se liant aux mycotoxines, avant la détection du signal.

9. Procédé selon l'une quelconque des revendications précédentes, **caractérisé en ce que** les mycotoxines sont choisies dans le groupe comprenant les aflatoxines, les ochratoxines, les alcaloïdes d'ergots, la patuline et/ou les toxines de Fusarium, par exemple choisies dans le groupe comprenant le désoxynivalénol, le nivalénol, la zéaralénone, la toxine T-2, la toxine HT-2 et/ou la fumonisine, de préférence choisies dans le groupe comprenant l'ochratoxine A, le désoxynivalénol, le nivalénol, la zéaralénone, la toxine T-2, la toxine HT-2, la fumonisine B1, la fumonisine B2 et/ou la fumonisine B3.

10. Procédé selon l'une quelconque des revendications précédentes, **caractérisé en ce que** la détection est réalisée sous la forme d'un immunoessai.

11. Kit pour la détection rapide de mycotoxines, **caractérisé en ce que** le kit contient au moins un guide d'ondes à couches minces comprenant une première couche guide d'ondes optiquement transparente (a) sur une seconde couche optiquement transparente (b), (b) présentant un indice de réfraction inférieur à (a) et des conjugués mycotoxine-albumine de sérum bovin étant immobilisés de manière séparée dans l'espace sur la couche guide d'ondes (a) en tant qu'élément de reconnaissance chimique ou biochimique pour un partenaire de liaison se liant aux mycotoxines.

12. Kit selon la revendication 11, **caractérisé en ce que** le kit contient un réactif comprenant de préférence des partenaires de liaison se liant aux mycotoxines marqués par fluorescence.

13. Utilisation d'un kit selon l'une quelconque des revendications précédentes pour la détection rapide de mycotoxines.
